# EUROPEAN PATENT SPECIFICATION

(11) **EP 1 964 582 B1**
(45) Date of publication and mention of the grant of the patent: **25.03.2020**
(21) Application number: 06833452.3
(22) Date of filing: 28.11.2006
(51) Int. Cl.: A61L 27/38, A61L 27/56, A61L 27/58, A61L 31/14, D04H 3/02, D04H 3/16, D01D 5/00, D01F 6/62, D01F 6/84

(54) **BIOCOMPATIBLE MATERIAL HAVING BIOCOMPATIBLE NON-WOVEN NANO- OR MICRO-FIBER FABRIC PRODUCED BY ELECTROSPINNING METHOD**
BIOKOMPATIBLES MATERIAL MIT EINEM BIOKOMPATIBLEN VLIES AUS NANO- ODER MIKROFASERN, DAS DURCH ELEKTROSPIN-VERFAHREN HERGESTELLT WURDE
MATÉRIAU BIOCOMPATIBLE AYANT UNE STRUCTURE BIOCOMPATIBLE NON TISSÉE DE NANO- OU DE MICRO-FIBRES, PRODUIT PAR ÉLECTROFILAGE

(30) Priority: 02.12.2005 JP 2005348799
(43) Date of publication of application: 03.09.2008
(73) Proprietor: Sunstar Suisse SA, 1024 Ecublens (CH)
(72) Inventor: KITA, Kazuyoshi, Takatsuki-shi, Osaka 569-1195 (JP); KATSURAGI, Yasuhiro, Takatsuki-shi, Osaka 569-1195 (JP); TAKEMURA, Akane, Takatsuki-shi, Osaka 569-1195 (JP)
(74) Representative: Gill Jennings & Every LLP
(86) International application number: PCT/JP2006/323648
(87) International publication number: WO 2007/063820

(56) References cited:
- WO-A1-90/07308
- WO-A1-92/15340
- WO-A1-2004/087232
- WO-A2-03/024297
- WO-A2-03/024297
- WO-A2-2004/000915
- WO-A2-2006/068809
- JP-A- 2004 024 706
- JP-A- 2004 321 484
- JP-A- 2005 290 610
- US-A- 4 961 707
- US-A1- 2002 090 725
- US-A1- 2002 090 725
- SHIN M ET AL: "In vivo bone tissue engineering using mesenchymal stem cells on a novel electrospun nanofibrous scaffold", TISSUE ENGINEERING, LARCHMONT, NY, US, vol. 10, no. 1-2, 1 January 2004 (2004-01-01), pages 33-41, XP002389116, ISSN: 1076-3279, DOI: DOI:10.1089/107632704322791673
- LI W.J. ET AL: 'Multilineage differentiation of human mesenchymalstem cells in a three-dimensional nanofibrous scaffold' BIOMATERIALS vol. 26, no. 25, 2005, pages 5158 - 5166, XP003013672
- LI W.J. ET AL: 'Three-dimensional nanofibrous scaffold forcarilage tissue engineering using human mesenchymal stem cells' BIOMATERIALS vol. 26, no. 6, 2005, pages 599 - 609, XP003013673
- KURTIS B. ET AL: 'Effect of polylactide/glycolide (PLGA) membranes loaded with metronidazole on periodontal regeneration following guided tissue regeneration in dogs' JOURNAL OF PERIODONTOLOGY vol. 73, no. 7, 2002, pages 694 - 700, XP003013674
- HARADA H. ET AL: 'FGF10 maintains stem cell compartment indeveloping mouse incisors' DEVELOPMENT vol. 129, no. 6, 2002, pages 1533 - 1541, XP003013675
- WON-SEOK SONG ET AL: "The effects of a bioabsorbable barrier membrane containing safflower seed extracts on periodontal healing of 1-wall intrabony defects in beagle dogs", JOURNAL OF PERIODONTOLOGY, AMERICAN ACADEMY OF PERIODONTOLOGY, CHICAGO, IL, US, vol. 76, no. 1, 1 January 2005 (2005-01-01), pages 22-33, XP008178203, ISSN: 0022-3492, DOI: 10.1902/JOP.2005.76.1.22
- SHIN M ET AL: "In vivo bone tissue engineering using mesenchymal stem cells on a novel electrospun nanofibrous scaffold", TISSUE ENGINEERING, LARCHMONT, NY, US, vol. 10, no. 1-2, 1 January 2004 (2004-01-01), pages 33-41, XP002389116, ISSN: 1076-3279, DOI: 10.1089/107632704322791673

## Description

Biocompatible material having biocompatible non-woven nano- or micro-fiber fabric produced by electrospinning method.

### TECHNICAL FIELD

The present invention relates to a biocompatible material comprising a biocompatible nano- or microfiber nonwoven fabric produced using an electrospinning method.

### BACKGROUND ART

Biocompatible materials such as guided tissue regeneration membranes (GTR membranes) or guided bone regeneration membranes (GBR membranes), have been used in the medical field for the purposes of anagenesis or osteoanagenesis, particularly in dentistry or oral surgery. As disclosed in Patent Document 1, the biocompatible materials have conventionally been made of a copolymer or a homopolymer of a glycolic acid, a lactic acid or a caprolactone; or a mixture of them. Various kinds of biocompatible materials have been reported, including a material containing a microporous polymer-ceramic material (Patent Document 2); a sponge-like material made of a polycondensation product of a lactic acid, a glycolic acid, a caprolactone, or a copolymer of those (Patent Document 3); a bioabsorbable membrane with fully-permeable pores, made by molding an emulsion of a membrane-forming polymer material (Patent Document 4); a multilayer membrane consisting of collagen II (Patent Document 5); and a material having a porous sheet-like structure made of a high-molecular blend material selected from a homopolymer or a copolymer of a L-lactic acid, a DL-lactic acid, a glycolic acid, or a e-caprolactone, wherein the pores are 1 to 50µm in diameter, 5 to 95% in porosity, and 50 to 500pm in thickness (Patent Document 6). Further, Patent Documents 7 and 8 disclose a porous scaffold material serving to regenerate defective parts of living tissue, which ensures acceleration of cell growth and an increase in adhesion to stem cells.

Those materials and membranes have been typically produced using a method in which a polymer is dissolved in a solvent and the solution is evaporated to obtain a membrane product, or a method in which a polymer emulsion is applied to a shape retention material and the dry membrane product is peeled from the material. However, perforation of the membranes needs to be carried out using lasers or the like after the membranes are formed, which complicates manufacturing.
(Patent Document 1) Japanese Unexamined Patent Publication No. H06-504702
(Patent Document 2) Japanese Unexamined Patent Publication No. H06-319794
(Patent Document 3) Japanese Unexamined Patent Publication No. H10-234844
(Patent Document 4) Japanese Unexamined Patent Publication No. H11-80415 (Patent Document 5) Japanese Unexamined Patent Publication No. 2001-519210
(Patent Document 6) Japanese Unexamined Patent Publication No. 2002-85547 (Patent Document 7) Japanese Unexamined Patent Publication No. 2005-110709
(Patent Document 8) Japanese Unexamined Patent Publication No. 2004-298544
WO03/024297A2 discloses cell storage and delivery systems and methods for storing and delivering viable cells to a mammal.
US2002/0090725A1 discloses formation and use of electro-processed collagen, including use as an extracellular matric and, together with cells, its use in forming engineered tissue.
Shin et al., "in vivo bone tissue engineering using mesenchymal stem cels on a novel electrospun nanofibrous scaffold", Tissue Engineering, Vol. 10, No. 1/2 2004, discloses a study to assess bone formation from mesenchymal stem cells on a nanofibrous scaffold in a rat model.
Won-Seok Song et al., "The effects of a bioabsorbable barrier membrane containing safflower seed extracts on periodontal healing of 1-wall intrabony defects in beagle dogs", J. Periodontology, Vol. 74, No. 1, 1 January 2005, pages 22-33, discloses a study that suggests that surgical application of polyactide glycolic acid non-woven membrane could promote the regeneration of alveolar bone and cementum in intrabony periodontal defects.
JP2004024706 discloses a sheet for viable tissue regeneration.

### DISCLOSURE OF THE INVENTION

### Technical Problem

Anagenesis and osteoanagenesis operations are carried out using various factors to induce or facilitate the regeneration. Therefore, biocompatible materials for use in anagenesis or osteoanagenesis purposes, such as a guided tissue regeneration membrane (GTR membrane), a guided bone regeneration membrane (GBR membrane), an anagenesis scaffold material that can contain stem cells or growth factors (e.g. a sheet material, a patch material, or a compensation material serving as an anagenesis scaffold material) need to be porous to allow transportation of those factors. In this view, the present invention is aimed at providing a microporous biocompatible material such as a microporous guided tissue regeneration membrane (GTR membrane), a guided bone regeneration membrane (GBR membrane), a sheet material, a patch material, or a compensation material. The present invention also provides a method for easily manufacturing such biocompatible materials.

### Technical Solution

As a result of intensive studies to attain the foregoing object, the inventors of the present invention found that a biocompatible material comprising biocompatible nano- or microfiber nonwoven fabric, such as a guided tissue regeneration membrane or a guided bone regeneration membrane, can be easily produced using an electrospinning method. Based on this finding, the inventors completed the present invention.

Specifically, the present disclosure related to the biocompatible material according to claim 1.

In the present specification, "biocompatibility" designates a characteristic of not causing excessive harmful effect to living organisms. "Nano- or microfiber" designates a fine fiber such as a nanofiber or a microfiber. A nanofiber designates a fiber 1µm or less in diameter. A microfiber designates a fiber not less than 1µm and not more than 1mm in diameter. The diameter of the nano- or microfiber preferably ranges from 10nm to 500pm, more preferably 100nm to 100pm. Further, a nano- or microfiber nonwoven fabric designates a nonwoven fabric made of a nano- or microfiber, including a nonwoven fabric made of fibers whose average diameter is on a nanometer or micrometer scale. The content of the nano- or microfibers in a nonwoven fabric is preferably not less than 50 wt%. Note that the diameter of the nano- or micro-fiber is calculated by carrying out a SEM measurement and then finding an average diameter using image processing or the like.

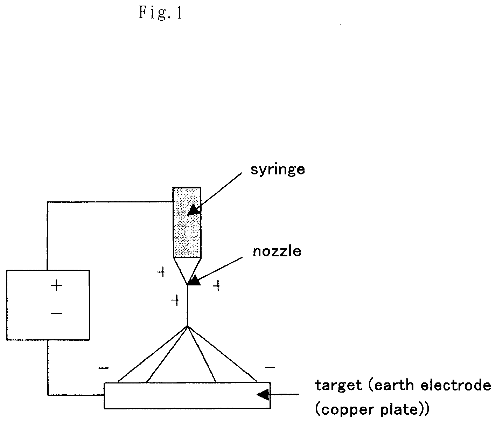

An electrospinning method generally adopts solution-spinning, which is a publicly known fabrication method for nano- or microfiber nonwoven fabric. FIG. 1 shows a general mechanism of the electrospinning method. A positive high voltage is applied to the spinning solution (polymer solution). The charged polymer solution becomes sharp, conical-shaped drops in the positive electrode. The drops then narrow further, and scatter toward the ground or the negative electrode. The splashes of the solution droplets are vaporized as they fly in the air, and the polymers are converted into fibers (nanofibers). The resulting fibers are collected in the negative electrode, thereby obtaining a nano- or microfiber nonwoven fabric. This theory is described in Kakou-gijyutsu (processing technology) vol. 40, No.2 (2005) 101-103, Kakou-gijyutsu vol.40, No.3 (2005) 167-171, or in Kakou-gijyutsu, vol. 40, No.4 (2005) 272-275. In the present invention, the electrospinning method includes all fabrication methods based on this theory.

The following describes a typical example of the electrospinning method according to the present invention.

In the electrospinning method, the spinning solution is loaded with a high voltage. Therefore, it is convenient to use a high-voltage transformer assembly. The voltage is generally not more than 100kV, preferably within a range from 1 to 40kV.

The container of the spinning solution may be a syringe, pipette, or a capillary that is generally made of polypropylene or glass. An appropriate capacity of the container depends on the shape, size, and thickness of the target nano- or microfiber nonwoven fabric.

The container is attached to the nozzle (e.g. an injector needle), which is preferably made of a conductive material. The nozzle is connected to the positive electrode. The nozzle exit is preferably round-shaped, but may have a different shape depending on the target nanofiber. It is possible to emit a plurality of fibers from the nozzle. The nozzle is connected to the positive electrode.

The nano- or microfiber emitted from the nozzle is deposited on the surface of the target, thereby forming a nonwoven fabric. The target is generally a plate, a bar, a wire mesh, a specifically-molded three-dimensional object etc. made of copper, stainless steel or the like. To ease the collection of the nonwoven fabric, the nonwoven fabric may be grown, for example, on an aluminum foil or the like placed on the target. For the sake of better homogenization, the nonwoven fabric may be formed on a rotating plate-shaped target or on a rolling bar-shaped target. Further, a belt conveyor target or a target containing a roller bed is useful to obtain a large nonwoven fabric. For example, to form a three-dimensional fabric, the fabrication is performed using a shifter capable of rotating the object at a certain angle. With this shifter, a table movable in the X, Y and Z directions and a table for keeping the fabrication surface in a particular direction (e.g. horizontal direction) are rotated at a certain angle. Fabrication is then carried out by shifting the target or the nozzle to an appropriate position to form the desired shape. However, the present invention is not limited to this method. The target is connected to the earth of the high-voltage transformer assembly.

One possible arrangement in this method is that a syringe with a nozzle is vertically disposed, and the target is placed right under the syringe. However, in this arrangement, the droplets from the nozzle also land in the target. To avoid this, it is preferable that the syringe etc. be arranged so that the nano- or microfiber is obliquely (e.g., at a 30 to 45° angle) ejected, and that the target be positioned to receive the obliquely-ejected nano- or microfiber, allowing the fiber to be deposited thereon. The distance from the nozzle head to the target is generally 5 to 70cm, preferably 5 to 30cm.

Examples of the material of the nano- or microfiber nonwoven fabric contained in the biocompatible material of the present invention is a homopolymer or copolymer of at least one kind selected from the group consisting of lactic acid, glycolic acid or a mixture of those polymers.

The weight-average molecular weight of the polymer is not limited as long as the polymer can be converted into a nano- or microfiber with the electrospinning method. The weight-average molecular weight of the polymer to be used is generally 100,000 to 10 million, preferably 100,000 to 1 million. Polymers with low molecular amounts may have difficulty in the conversion into a nano- or microfiber; however, such polymers can be secured for conversion by being mixed with other polymers. Further, to ensure the stiffness of the polymer chain, it is preferable to use crystalline polymers. The concentration of the polymer in the spinning solution is not particularly limited as long as the polymer solution is convertible into a nano- or microfiber with the electrospinning method. The concentration is generally 1 to 30 wt%, preferably 5 to 10 wt%. The solvent used for the spinning solution is selected based on its property for dissolving the polymer to be used. Examples of the solvent include hydrophilic organic solvents such as water, acetic acid, N,N-dimethylformamide, methanol, ethanol, acetone, or tetrahydrofuran; hydrophobic organic solvents such as chloroform, methylene chloride, dichloroethane, tetrachloroethane, trichloroethane, dibromomethane, or 1,1,3,3,3 hexafluoro-2-propanol; plasticizers such as acetyl citric acid ester, adipate ester, sebacic acid ester or phthalate ester; and mixtures of those.

Moreover, the spinning solution may contain various functional substances for inducing or promoting regeneration of tissue or bone so that the resulting nano- or microfiber nonwoven fabric have the properties derived from these functional substances. By thus incorporating the functional substances, it is not necessary to carry out an additional step for providing those properties after the biocompatible material is produced. Examples of the functional substance include bone and/or tissue growth factors such as PDGF (platelets-derived growth factor), IGF (insulin-like growth factor), BMP (bone neoplasia promoting factor), bFGF (basic fibroblast growth factor) or osteopontin; enamel proteins such as amelogenine or enamelin; biological materials such as albumin, globulin, chondroitin sulfate, fibronectin, fibrinogen or elastin; antibacterial agents including tetracyclines such as minocycline or doxycycline, macrolides such as clarithromycin or azithromycin; new quinolones such as levofloxacin, and ketolides such as telithromycin; nonsteroidal anti-inflammatory agents such as flurbiprofen, steroidal anti-inflammatory agents such as dexamethasone; natural products such as azulene; and medicinal agents including bone resorption inhibitors such as bisphosphonate. Note that these functional substances may have a gel form to be applied to the produced nano- or microfiber nonwoven fabric. Otherwise, insofar as their properties are not impaired, the medicines may be dissolved in the solvent together with the polymer when preparing a spinning solution, thereby preparing a medicated spinning solution. By subjecting this medicated spinning solution to electrospinning, a nonwoven fabric with medicated fibers is obtained.

The spinning solution may contain other additives generally used for the electrospinning method, for example, a surfactant, or an electrolyte such as lithium chloride.

The biocompatible material according to the present invention contains a biocompatible nano- or microfiber nonwoven fabric produced by the electrospinning method. According to the method of the present invention, the polymer solution is repeatedly sprayed to the surface of the same target so that nanofibers are deposited thereon. With this method, the present invention manufactures a superior stereoscopic structure (solid).

A dental biocompatible material designates a biocompatible material used in the dental field. Examples of the dental biocompatible material include anagenesis or osteoanagenesis materials, such as a guided tissue regeneration membrane (GTR membrane), a guided bone regeneration membrane (GBR membrane) or an anagenesis scaffold material that can contain stem cells or growth factors (e.g. a sheet material, a patch material, or a compensation material serving as an anagenesis scaffold material). The stem cells or growth factors may be contained inside the biocompatible material or on the surface of the biocompatible material. Oral biocompatible material designates a biocompatible material used in the oral surgery field. Examples of the oral biocompatible material include antiadhesive agents for preventing adhesion after surgery; anagenesis or osteoanagenesis materials, such as a guided tissue regeneration membrane (GTR membrane), a guided bone regeneration membrane (GBR membrane) or an anagenesis scaffold material that can contain stem cells or growth factors (e.g. a sheet material, a patch material, or a compensation material serving as an anagenesis scaffold material). The biocompatible material of the present disclosure is used for the dental treatment of defective alveaolar bone according to claim 1 and to the claims 2 to 7 all dependent on claim 1. Further, the biocompatible material is preferably biodegradable. This increases the security of usage in living organisms, and does not require the removal of the membrane after the biocompatible material is applied to the affected part.

The thickness of the biocompatible material according to the present invention is not particularly limited. In the case of GTR membrane or GBR membrane, the material is generally 100 to 1,000pm thick, preferably 200 to 700pm thick. In the case of anagenesis scaffold material for stem cells, the size of the material preferably corresponds to the affected part to which the scaffold material is applied. The material can be cut into an appropriate size for the affected part before application.

The porosity of the biocompatible material of the present invention is preferably 5 to 95%. The pore distribution can be adjusted by the concentration of polymers, molecular weight, or application frequency.

The appropriate shape and size of the biocompatible material of the present invention is determined according to the form and size of the affected part.

The biocompatible material is given appropriate shape, size, pore system, and thickness by adjusting the condition of the electrospinning method (e.g., the target rotation speed). To manufacture a biocompatible material of a complicated shape, the biocompatible material is processed into a desired shape or structure after the electrospinning.

### [Effect of the Invention]

Using the electrospinning method, the present invention easily fabricates a nano- or microfiber nonwoven fabric having voids, which serves as a superior biocompatible material.

### BRIEF DESCRIPTION OF THE DRAWINGS

FIG. 1 is a schematic diagram illustrating an apparatus used in Examples 1 to 3.
FIG. 2 is an optical microscope image of a nano fiber nonwoven fabric produced in Example 2. A grade of the scale is 1µm.
FIG. 3 is a schematic diagram illustrating an affected part of a dog model of Example 4, showing the alveolar bone and the position of the nano fiber nonwoven fabric (GTR membrane) applied to the alveolar bone. The thick, black line denotes the GTR membrane. The figure shows the state after the operation in which the regenerated epithelial tissue is covering the nonwoven fabric, and the new bone is appearing mainly along the direction indicated by the arrow.
FIG. 4 is a schematic diagram illustrating an affected part of a dog model of Example 6, showing the defective born part and the position of the nonwoven fabric of the present invention. The thick, black line denotes the GTR membrane. The figure shows the state after the operation in which the regenerated epithelial tissue is covering the nonwoven fabric, and the new bone is appearing mainly along the direction indicated by the arrow.

### BEST MODE FOR CARRYING OUT THE INVENTION

The present invention will be more specifically described with reference to the following Examples; however, the present invention is not limited to these Examples.

### Examples

### Example 1

1-polylactic acid (weight average molecular weight: approximately 110,000) was dissolved in chloroform to obtain a 7 wt% solution. The solution is poured in a syringe shown in FIG.1 to be subjected to electrospinning at 12kV. A nanofiber nonwoven fabric was thus obtained. The distance from the nozzle tip to the target was 15cm. The mean diameter of the nanofibers in the resulting nanofiber nonwoven fabric was approximately 500nm. Thickness of the nonwoven fabric was approximately 40pm.

### Example 2

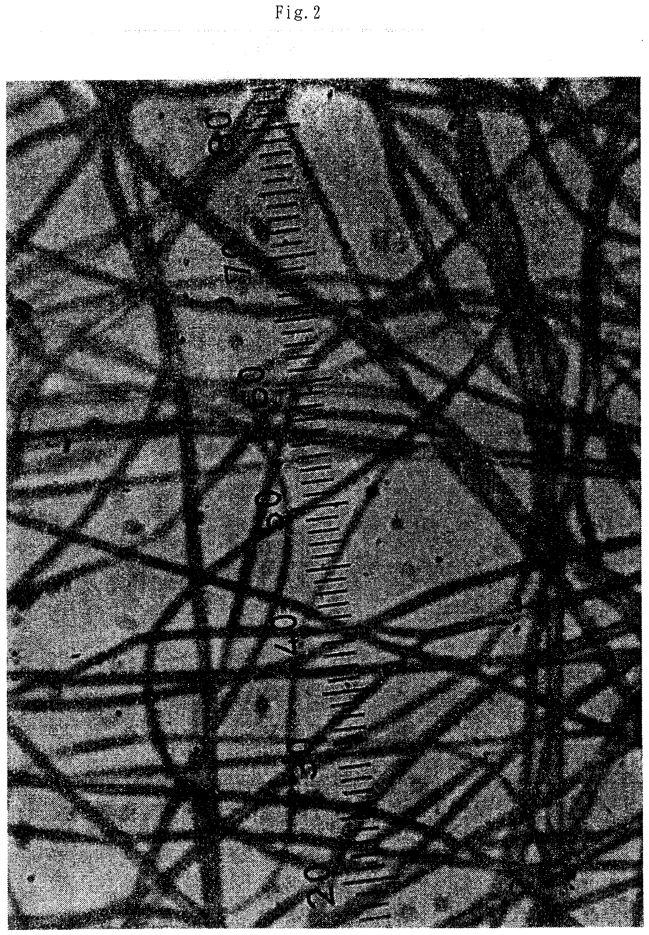

A copolymer of d,l-polylactic acid and glycolic acid (composition ratio: 10:90, weight average molecular weight: approximately 750,000) was dissolved in 1,1,3,3,3-hexafluoro-2-propanol to obtain a 10 wt% solution. The solution was subjected to electrospinning at 2kV. A nanofiber nonwoven fabric was thus obtained. The distance from the nozzle tip to the target was 15cm. The mean diameter of the nanofibers in the resulting nanofiber nonwoven fabric was approximately 1,000nm. The thickness of the nonwoven fabric was approximately 280pm. FIG. 2 shows a microscope image of this nonwoven fabric.

### Example 3

Chitosan approximately 500 mPa·s in viscosity (0.5 % acetic acid) was dissolved in a 90% acetic acid to obtain a 7 wt% solution. The solution was subjected to electrospinning at 4kV. A nanofiber nonwoven fabric was thus obtained. The distance from the nozzle tip to the target was 5cm. The mean diameter of the nanofibers in the resulting nanofiber nonwoven fabric was approximately 150nm.

### Example 4: GTR membrane

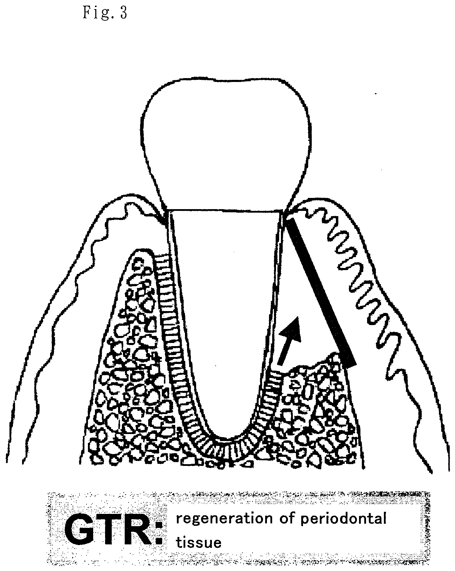

The right and left 4th bicuspid teeth in the lower jaw of a beagle were pulled out, leaving the beagle an artificial 2-walled intrabony periodontal defect. The affected part was exposed, and then the beagle model was treated with scaling and root planing. The nanofiber nonwoven fabric (GTR membrane) obtained in Example 1 was sterilized and applied to the affected part, while ensuring enough space for allowing the alveolar bone and the periodontal membrane to recover. Then, the gingiva was placed back in and banded with an absorbable surgical suture. 1 month and 3 months after the application of the nanofiber nonwoven fabric to the affected part, a pathologic tissue sample was made to observe the change of the periodontal tissue form, using picture processing. The bone density was also examined with a soft X-ray image. FIG.3 shows a schematic diagram of the GTR membrane applied to the affected part.

Further, the same operation was performed with respect to the same dog model using a membrane comprising experimental polylactic acid, and the progress was observed.

With regard to the nano- or microfiber nonwoven fabric, 3 months after the application, bias filling of a thick collagen fiber bundle was seen in the newly-erupted periodontal ligament, and new attachment in which the fiber stumps are embedded in the new cementum or the new alveolar bone was also observed. The bone density of the part having the defective bone recovered to approximately 10% after a month, and recovered to approximately 20% after 3 months. In contrast, in the dog model using the membrane of polylactic acid, even though the new adhesion was observed in a part of the tissue after 3 months, the bone density of the part having the defective bone stayed the same as that of application time both after a month and after 3 months, and no improvement was seen in the transmissive image of the bone defect part. This showed that the nanofiber nonwoven fabric was useful for periodontal tissue regeneration.

### Example 5: GBR membrane

A copolymer of d,l-polylactic acid and glycolic acid (composition ratio 75:25, weight average molecular weight: approximately 6,000) was dissolved in a mixed solution of tetrahydrofuran and N,N-dimethylformamide (volume ratio 1:1) to obtain a 5 wt% solution. The solution was subjected to electrospinning at 18kV. A nanofiber nonwoven fabric was thus obtained. The distance from the nozzle tip to the target was 20cm. The mean diameter of the nanofibers in the resulting nanofiber nonwoven fabric was approximately 700nm. A 0.1 wt% solution obtained by dissolving bFGF (product of Merck) in an amino acid buffer solution was dropped into this nonwoven fabric. The fabric was then freeze-dried to obtain a bFGF-containing nonwoven fabric.

Three rats (30 weeks old) were given a general anesthetic. After cutting open the right and left palatine portions of the upper jaw molar, the entire palatine membrane layer was peeled off. In order to distinguish the existing bone from new bone, nylon yarn (registered trademark, general name: polyamide yarn) was placed on the born-surfaces of the right palatine portion and the left palatine portion. In the left palatine portion, the bFGF-containing nonwoven fabric thus produced above was placed on the yarn to fit into the palatine groove. In the right palatine portion, a bFGF-free nonwoven fabric otherwise similar to the bFGF-containing nonwoven fabric was placed on the yarn to fit into the palatine groove. Thereafter, the both palatine portions were sutured. After 6 weeks of observation, the affected part was observed histopathologically.

The average height of the new bones in the right palatine portion where the bFGF-free nonwoven fabric was applied was 41pm. This shows that the nonwoven fabric is effective for the regeneration of new bones. Further, the average height of the new bones in the left palatine portion where the bFGF-containing nonwoven fabric was applied was 98pm. This shows that the bFGF-containing nonwoven fabric can further facilitate regeneration.

### Example 6: GBR membrane

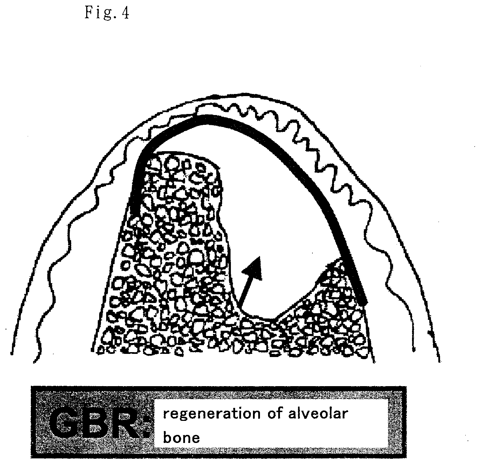

The right and left 4th bicuspid teeth in the lower jaw of a beagle were pulled out, leaving the beagle an artificial 2-walled intrabony periodontal defect. The affected part was exposed, and then the left defective portion was covered with the bFGF-containing nonwoven fabric produced in Example 5, and the right defective portion was covered with a bFGF-free nonwoven fabric otherwise similar to the bFGF-containing nonwoven fabric (FIG. 4). The two defective portions were covered with a gingival tissue. 1 month and 3 months after the application to the affected part, a pathologic tissue sample was made. The bone density was also examined with a soft X-ray image. FIG. 4 shows a schematic diagram of the GTR membrane applied to the affected part.

The analysis showed that the bone densities of both of the right and left defective bone portions increased. The bone density recovery rate of the left defective bone portion was 50% higher than that of the right defective bone portion, both after 1 month and 3 months. This result showed that the bFGF-free nonwoven fabric is useful to increase the alveolar bone density, and that the bFGF-containing nonwoven fabric further facilitates the increase in alveolar bone density.

## Claims

1. A biocompatible material comprising a biocompatible nano- or microfibre non-woven fabric produced by electrospinning for use in the treatment of defective alveolar bone,
a material of the non-woven fabric being a homopolymer or copolymer of at least one compound selected from the group of consisting of lactic acid and glycolic acid, or a mixture of those polymers,
the non-woven fabric containing a growth factor, wherein the growth factor is bFGF,
the biocompatible material being freeze dried.

2. The biocompatible material according to claim 1, wherein the electrospinning method is conducted by using a spinning solution that contains the growth factor.

3. The biocompatible material according to claim 1 or 2, wherien the growth factor is contained in the nonwoven fabric through steps as follows:
dropping a solution obtained by dissolving the growth factor into the nonwoven fabric, and
freeze-drying the nonwoven fabric.

4. The biocompatible material according to any preceding claim, wherein the material of the nonwoven fabric is a copolymer of d,l-polylactic acid and glycolic acid.

5. The biocompatible material according to any preceding claim, wherein the biocompatible material is a guided bone regeneration membrane (GBR membrane).

6. The biocompatible material according to any one of claims 1 to 5, wherein the nano- or microfibre has a fibre diameter of 10 nm to 500 µm.

7. The biocompatible material according to any one of claims 1 to 6, wherein the biocompatible material has a thickness of 100 to 1000 µm.

## Patentansprüche

1. Biokompatibles Material, umfassend einen biokompatiblen Nano- oder Mikrofaservliesstoff, der durch Elektrospinnen hergestellt ist, zur Verwendung in der Behandlung eines defekten Alveolarknochens,
wobei ein Material des Vliesstoffes ein Homopolymer oder Copolymer von mindestens einer Verbindung, ausgewählt aus der Gruppe, bestehend aus Milchsäure und Glycolsäure, oder ein Gemisch aus diesen Polymeren ist,
der Vliesstoff einen Wachstumsfaktor enthält, wobei der Wachstumsfaktor bFGF ist,
wobei das biokompatible Material gefriergetrocknet ist.

2. Biokompatibles Material nach Anspruch 1, wobei das Elektrospinnverfahren durch Verwendung einer Spinnlösung, die den Wachstumsfaktor enthält, durchgeführt wird.

3. Biokompatibles Material nach Anspruch 1 oder 2, wobei der Wachstumsfaktor in dem Vliesstoff durch folgende Schritte enthalten ist:
Fallenlassen einer Lösung, erhalten durch Lösen des Wachstumsfaktors, in den Vliesstoff, and
gefriertrocknen des Vliessstoffes.

4. Biokompatibles Material nach einem vorhergehenden Anspruch, wobei das Material des Vliesstoffes ein Copolymer aus d,l-Polymilchsäure und Glycolsäure ist.

5. Biokompatibles Material nach einem vorhergehenden Anspruch, wobei das biokompatible Material eine geführte Knochenregenerationsmembran (GBR-Membran) ist.

6. Biokompatibles Material nach einem der Ansprüche 1 bis 5, wobei die Nano- oder Mikrofaser einen Fasserdurchmesser von 10 nm bis 500 µm aufweist.

7. Biokompatibles Material nach einem der Ansprüche 1 bis 6, wobei das biokompatible Material eine Dicke von 100 bis 1000 µm aufweist.

## Revendications

1. Matériel biocompatible comprenant un tissu non tissé biocompatible de nano- ou microfibres produit par électrofilage pour une utilisation dans le traitement d'os alvéolaire défectueux,
un matériel du tissu non tissé étant un homopolymère ou un copolymère d'au moins un composé choisi parmi le groupe constitué d'acide lactique et d'acide glycolique, ou un mélange de ces polymères,
le tissu non tissé contenant un facteur de croissance, le facteur de croissance étant bFGF,
le matériel biocompatible étant lyophilisé.

2. Matériel biocompatible selon la revendication 1, dans lequel le procédé d'électrofilage est effectué en utilisant une solution de filage qui contient le facteur de croissance.

3. Matériel biocompatible selon la revendication 1 ou 2, dans lequel le facteur de croissance est contenu dans le tissu non tissé par les étapes suivantes consistant à :
déposer une solution obtenue en dissolvant le facteur de croissance dans le tissu non tissé, et
lyophiliser le tissu non tissé.

4. Matériel biocompatible selon l'une quelconque des revendications précédentes, dans lequel le matériel du tissu non tissé est un copolymère d'acide d,l-polylactique et d'acide glycolique.

5. Matériel biocompatible selon l'une quelconque des revendications précédentes, dans lequel le matériel biocompatible est une membrane pour régénération osseuse guidée (membrane GBR).

6. Matériel biocompatible selon l'une quelconque des revendications 1 à 5, dans lequel la nano- ou microfibre a un diamètre de fibre de 10 nm à 500 µm.

7. Matériel biocompatible selon l'une quelconque des revendications 1 à 6, dans lequel le matériel biocompatible a une épaisseur de 100 à 1 000 µm.
